# EUROPÄISCHE PATENTSCHRIFT

(11) **EP 1 393 807 B1**
(45) Veröffentlichungstag und Bekanntmachung des Hinweises auf die Patenterteilung: **02.11.2005**
(21) Anmeldenummer: 03014901.7
(22) Anmeldetag: 01.07.2003
(51) Int. Cl.: B01L 3/00

(54) **Testvorrichtung zur Untersuchung einer biologischen Probenflüssigkeit**
Testing device for examining a biological sample
Dispositif d' essai pour l'analyse d' un échantillon biologique fluide

(30) Priorität: 31.07.2002 DE 10234819
(43) Veröffentlichungstag der Anmeldung: 03.03.2004
(73) Patentinhaber: Roche Diagnostics GmbH, 68305 Mannheim (DE); F. HOFFMANN-LA ROCHE AG, 4070 Basel (CH)
(72) Erfinder: Marquant, Michael, 68309 Mannheim (DE); Unkrig, Volker, Dr., 68526 Ladenburg (DE); Hindelang, Fritz, Dr., 67316 Carlsberg (DE)
(74) Vertreter: Pfiz, Thomas

(56) Entgegenhaltungen:
- WO-A-01/25137
- WO-A-01/89695
- DE-A- 19 933 458

## Beschreibung

Die Erfindung betrifft eine Testvorrichtung zur Untersuchung einer insbesondere biologischen Probenflüssigkeit mit einem aus mehreren Flachmateriallagen gebildeten Verbundkörper und mindestens einem in dem Verbundkörper angeordneten Probenkanal zum Transport der Probenflüssigkeit von einer Aufgabestelle zu einer Messstelle. Die Erfindung betrifft weiter ein Verfahren zur Herstellung einer solchen Testvorrichtung und deren bevorzugte Verwendungen.

Aus der WO 99/29429 ist ein analytisches Wegwerf-Testelement zur Bestimmung eines Analyten in einer Flüssigkeit bekannt, mit dem unter Verwendung minimaler Probenvolumina eine räumliche Trennung von Probenaufgabestelle und Detektionszone möglich ist. Dafür ist ein einzelner zum kapillaren Flüssigkeitstransport befähigter Kanal vorgesehen, dessen Geometrie von einer Zwischenschicht bestimmt ist, während ein Träger und eine Abdeckung aus Flachmaterial für eine untere und obere Begrenzung sorgen. Für solche diagnostische Systeme ist es essentiell, dass die Richtigkeit der vom System erhobenen Messergebnisse zu jedem Zeitpunkt sichergestellt ist. Mangelhafte Wartung von Geräten, falsche Lagerung von Reagenzien, Teststreifen oder Systemflüssigkeiten, Überschreitung von Verfallsdaten oder fehlerhafte Handhabung durch den Benutzer sind nur einige Gründe, die in der Praxis zu fehlerhaften Messergebnissen führen können. Für die Qualitätskontrolle von Testträgern sind daher Kontrollen üblich, bei denen Flüssigreagenzien anstelle von Probenmaterial auf den Teststreifen aufgetragen werden, um in vorgegebenen Sollbereichen liegende Messergebnisse zu erzeugen. Ein Nachteil dieser Methode ist, dass der überprüfte Testträger dadurch verbraucht ist und für eine Vermessung des eigentlichen Probenguts nicht mehr zur Verfügung steht. Mit dieser Methode ist also nur eine stichprobenartige Überprüfung einer größeren Anzahl von Testträgern möglich.

Um diesen Nachteil zu überwinden, ist es aus der US-A 5,591,403 an sich bekannt, mehrere in einer Ebene liegende Probenkanäle vorzusehen, die alle mit derselben Flüssigprobe gefüllt werden, wobei mindestens ein Kanal eine Kontrollsubstanz enthält. Ein Nachteil derartiger planarer Kanalgeometrien liegt darin, dass die Fertigung aufwendig und kostenintensiv ist. Insbesondere die Integration von unterschiedlichen Reagenzien in eng nebeneinander liegenden Kanälen unter strikter Trennung der einzelnen Prozesse ist nur schwierig zu verwirklichen.

Ausgehend hiervon liegt der Erfindung die Aufgabe zugrunde, die vorstehend beschriebenen Nachteile des Standes der Technik zu vermeiden und eine fertigungstechnisch günstige und zugleich kompaktere Bauform zu erreichen, mit welcher auch komplexere Testformate realisierbar sind. Weiter soll ein einfaches Herstellungsverfahren für solche Testvorrichtungen bzw. Testelemente angegeben werden.

Zur Lösung dieser Aufgabe wird die im Patentanspruch 1 bzw. 20 angegebene Merkmalskombination vorgeschlagen. Vorteilhafte Ausgestaltungen und Weiterbildungen der Erfindung ergeben sich aus den abhängigen Ansprüchen.

Der Kern der Erfindung liegt darin, ein Mehrfach-Testelement in gestapelter Anordnung mit übereinander liegenden verzweigungsfreien Probenkanälen vorzusehen. Dementsprechend wird erfindungsgemäß vorgeschlagen, dass der Verbundkörper mehrere zwischen Trägerlagen stapelförmig angeordnete Transportlagen zur Aufnahme jeweils eines zu einer zugeordneten Messstelle führenden Probenkanals aufweist. Damit ist es möglich, in einer einfachen, mehrfach wiederholten Schichtstruktur eine Mehrzahl von Probenkanälen in kompakter herstellungsfreundlicher Bauform übereinander anzuordnen. Dadurch werden auch deutliche Vorteile für die Verpackung und das Adaptieren in einem Messgerät erreicht. Weiterhin kann eine Verschleppung verschiedener Reagenzien während der Fertigung vermieden werden durch die Möglichkeit der zeitlichen und räumlichen Trennung der Applikation auf den verschiedenen Materiallagen, die erst später aufeinander montiert werden.

Besonders bevorzugt sind die Probenkanäle in Stapelrichtung der Transportlagen gesehen vorzugsweise fluchtend übereinander angeordnet, so dass auch die Probenaufgabe weiter vereinfacht wird. In einer vorteilhaften Ausführung ist es vorgesehen, dass die Probenkanäle als durchgehender Freiraum zwischen jeweils zwei gesonderten Teilstücken der Transportlagen freigehalten sind und insbesondere durch Zerteilen oder Ausstanzen der Transportlagen freigeschnitten sind.

Um eine Elektrodenanordnung im Bereich der Probenkanäle zu vereinfachen, sollten die Transportlagen aus einem elektrisch isolierenden Folienmaterial bestehen. Die Elektroden lassen sich vorteilhafterweise dadurch realisieren, dass die Trägerlagen an ihren den Transportlagen zugewandten Seiten mit einer Elektrodenschicht aus einem elektrisch leitfähigen Material geometrisch strukturiert oder vollfächig beschichtet sind. Eine weitere vorteilhafte Ausführung sieht hierbei vor, dass die beidseitig an den Transportlagen einander gegenüberliegenden Elektrodenschichten im Bereich der Messstellen ein Elektrodenpaar zur elektrochemischen Analyse einer Probeneigenschaft bilden. Hierfür ist es günstig, wenn die einander gegenüberliegenden Elektrodenschichten paarweise aus einem Edelmetall, vorzugsweise Gold, Platin oder Palladium als Messelektrode und einer Silber-Silberchlorid-Mischung als Gegen-Referenz-Elektrode bestehen.

Die Elektrodenschichten können zugleich zum Signalabgriff in einem Nachweisgerät dienen. Entsprechend ist es günstig, wenn die Elektrodenschichten eine randseitig über eine angrenzende Transportlage überstehende Anschlusspartie zur elektrischen Kontaktierung aufweisen. Eine weitere Verbesserung lässt sich dadurch erreichen, dass die Trägerlagen in einem Randbereich stufenförmig gegeneinander versetzt angeordnet sind.

Vorteilhafterweise sind die Transportlagen jeweils gegenüber mindestens einer angrenzenden Elektrodenschicht durch eine elektrisch isolierende Folienmaske getrennt, wobei die Folienmaske im Bereich des Probenkanals Durchbrüche zur Bildung von räumlich definierten Messfeldern an den vorgesehenen Messorten aufweist. Durch ein hydrophiles Material wird dabei zugleich gute und schnelle Befüllung mit einer wasserbasierenden Probenflüssigkeit erreicht.

Für den eigentlichen Nachweis des Analyten und zur Nachweiskontrolle ist es auf einfache Weise möglich, im Bereich der Messstellen von der Probenflüssigkeit aufnehmbare Reagenzien vorzugsweise als Trockensubstanz zu applizieren.

Vorteilhafterweise sind die Probenkanäle zwischen der Aufgabestelle und den jeweiligen Messstellen als Kapillarkanäle zum selbsttätigen Kapillartransport der Probenflüssigkeit ausgebildet.

Aufgrund der kompakten Anordnung können mehrere Probenkanäle zugleich durch Eintauchen in die aufzunehmende Flüssigprobe befüllt werden. Entsprechend sieht eine vorteilhafte Ausführung vor, dass die Aufgabestelle durch eine die Einlassöffnungen der Probenkanäle umfassende, in die Probenflüssigkeit eintauchbare Randzone des Verbundkörpers gebildet ist. Alternativ ist es auch möglich, dass die Aufgabestelle durch eine mit den Probenkanälen kommunizierende, mit einem Alliquot der Probenflüssigkeit befüllbare Ausnehmung des Verbundkörpers gebildet ist.

Um eine Durchmischung der gegebenenfalls mit unterschiedlichen Reagenzien beaufschlagten Probenflüssigkeit im Austrittsbereich der Probenkanäle zuverlässig zu vermeiden, ist es von Vorteil, wenn die Probenkanäle über quer zu den Flachmateriallagen verlaufende, im seitlichen Abstand voneinander angeordnete Entlüftungskanäle an der Außenseite des Verbundkörpers münden.

Zur Befüllungskontrolle der Probenkanäle sind vorteilhafterweise an mindestens einer Kontrollstelle geeignete Erfassungsmittel für die durchströmende Probenflüssigkeit vorgesehen. Dies lässt sich besonders einfach dadurch realisieren, dass mittels für den eigentlichen Nachweis vorhandener Elektroden während der Probenaufnahme die Änderung des Wechselstrom-Leitwerts gemessen wird.

Eine bevorzugte Ausführung zur Durchführung optischer Untersuchungen sieht vor, dass die Trägerlagen zumindest im Bereich der Messstellen transparente Messfenster bilden.

Herstellungstechnisch ist es von Vorteil, wenn der Verbundkörper aus stapelförmig miteinander verklebten Folienstreifen besteht.

Ein besonders bevorzugtes Verfahren zur Herstellung einer erfindungsgemäßen Testvorrichtung sieht vor, dass die Flachmateriallagen als bandförmige Rollenware von Rolle zu Rolle transportiert werden und im Zuge ihres Transports stapelförmig miteinander verklebt, gegebenenfalls mit Reagenzien versehen und nachfolgend zu Teststreifen zerteilt werden. Damit lässt sich ein kontinuierlicher Herstellungsprozess auf einfache Weise realisieren.

Ein weiterer Aspekt der Erfindung liegt in einer bevorzugten Verwendung der erfindungsgemäßen Mehrfach-Testvorrichtungen zur Bestimmung unterschiedlicher Parameter der Probenflüssigkeit in jeweils zugeordneten Probenkanälen. Eine weitere besonders vorteilhafte Verwendung sieht die gleichzeitige Erfassung von Messparametern der Probenflüssigkeit und von Kontrollparametern für die Gültigkeitsprüfung oder Kalibrierung der Messparameter in jeweils zugeordneten Probenkanälen vor.

Im Folgenden wird die Erfindung anhand der in der Zeichnung in schematischer Weise dargestellten Ausführungsbeispiele näher erläutert. Es zeigen:
- Fig. 1: eine als Teststreifen aus mehreren gestapelten Flachmateriallagen gebildete Testvorrichtung zur elektrochemischen Untersuchung einer Flüssigprobe wie Blut;
- Fig. 2: einen in die Vertikale gedrehten Schnitt entlang der Schnittlinie 2-2 der Fig. 1;
- Fig. 3: den lageweisen Aufbau des Teststreifens in der Draufsicht und Stirnseitenansicht beginnend mit der unteren Lage;
- Fig. 4: eine weitere Ausführungsform eines Teststreifens mit breitseitig angeordneten Entlüftungsöffnungen der Probenkanäle und stirnseitig überstehenden Kontaktlaschen;
- Fig. 5: einen Schnitt entlang der stufenförmigen Schnittlinie 5-5 der Fig. 4;
- Fig. 6: den lageweisen Aufbau des Teststreifens nach Fig. 5 in der Draufsicht beginnend mit der unteren Lage;
- Fig. 7 und 8: weitere Ausführungsformen von Teststreifen zur optischen Untersuchung der Probenflüssigkeit jeweils in der Draufsicht und in einem zentralen Längsschnitt.

Die in der Zeichnung dargestellten Teststreifen bestehen als Verbundkörper 10 im wesentlichen aus mehreren gegebenenfalls mit Elektrodenschichten 14, 16 versehenen Trägerlagen 12 und dazwischen angeordneten Transportlagen 18 zur Aufnahme jeweils eines Probenkanals 20, wobei eine elektrochemisch oder optisch zu untersuchende Probenflüssigkeit über die kapillaraktiven Probenkanäle von einer Aufgabestelle 22 zu jeweils mindestens einer Messzone bzw. Messstelle 24 transportierbar ist.

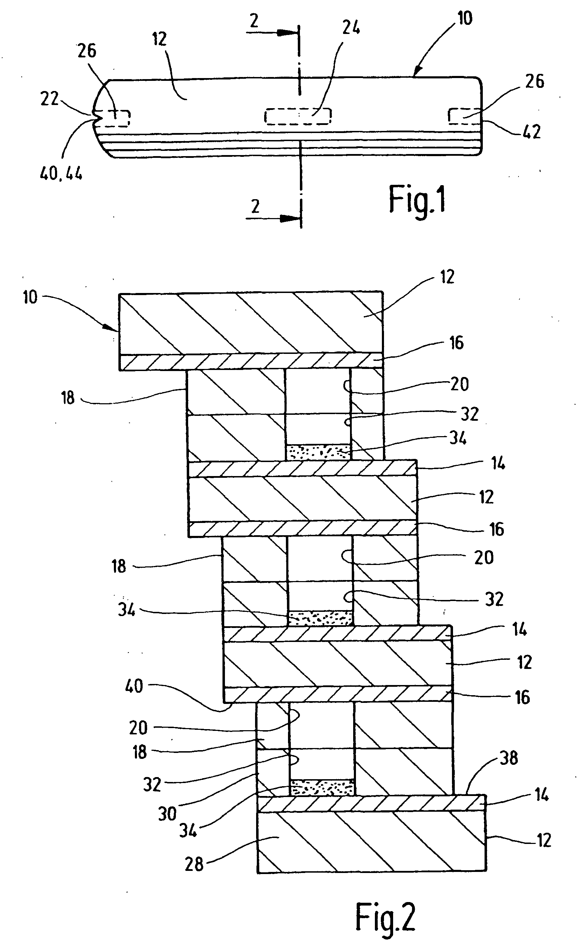

Wie in Fig. 2 gezeigt, sind die im Stapel der Transportlagen 18 jeweils einzeln freigehaltenen Probenkanäle 20 in Stapelrichtung, also quer zu den Materiallagen gesehen übereinander angeordnet, so dass eine besonders vorteilhafte Bauform erreicht wird. Zugleich ist es damit möglich, vollflächige nicht strukturierte Elektrodenschichten 14, 16 auf den die Probenkanäle 20 überdeckenden Trägerlagen 12 einzusetzen und eine verzweigende Kanalgeometrie zu vermeiden.

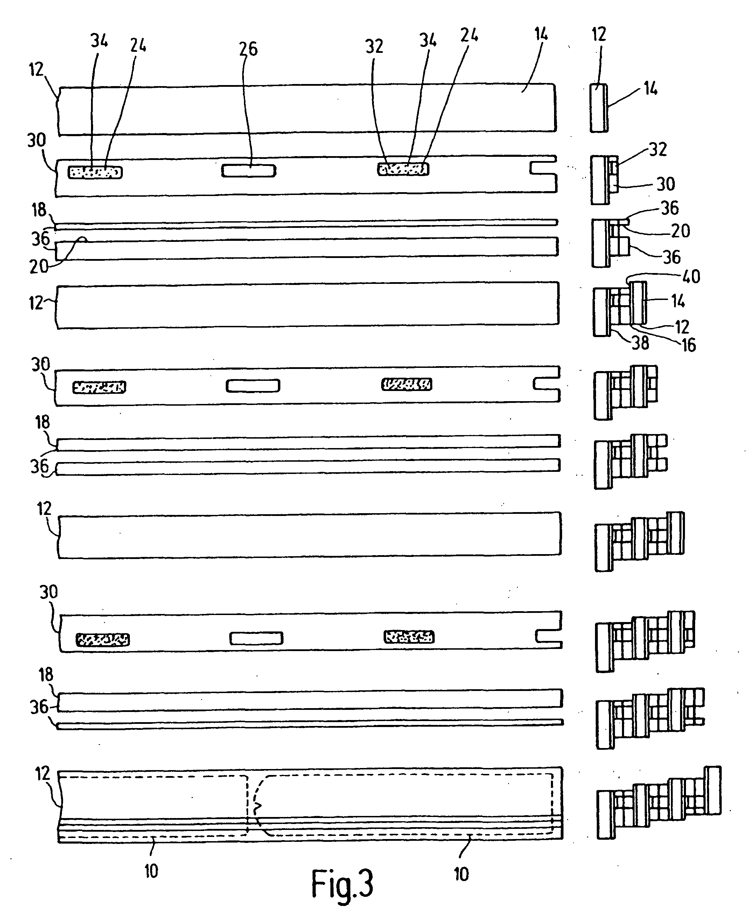

Anhand Fig. 3 wird der Aufbau der Teststreifen 10 als Abschnitte von miteinander verklebten Folienbändern näher erläutert. Eine mit einer Goldschicht 14 als Elektrodenfläche bedampfte Polyesterfolie 28 bildet die untere Trägerlage 12. Auf der Goldschicht 14 ist eine elektrisch isolierende Folienmaske 30 aufgeklebt, welche zugleich dauerhaft gute Benetzungseigenschaften für die Probenflüssigkeit besitzt. Die Folienmaske 30 ist in einem den Probenkanal bodenseitig begrenzenden Bereich an der zentralen Messstelle 24 und zusätzlichen ein- und austrittseitigen Kontrollstellen 26 mit Ausstanzungen bzw. Durchbrüchen 32 versehen, wodurch Elektrodenfelder der Goldschicht 14 an den entsprechenden Abschnitten des Probenkanals 20 begrenzt werden. Um eine elektrochemische Erfassung von Probeneigenschaften zu ermöglichen, sind an der Messstelle 24 in dem betreffenden Durchbruch 32 von der einströmenden Probenflüssigkeit aufnehmbare Reagenzien 34 auf der Goldschicht 14 dispensiert bzw. flächig aufgetragen und dann eingetrocknet. Die Kontrollstellen 26 dienen zur Befüllungskontrolle des Probenkanals 20 durch Messungen der von der durchströmenden Probenflüssigkeit beeinflussten elektrischen Leitfähigkeit.

Auf die Folienmaske 30 sind als erste Transportlage 18 zwei doppelseitig klebende Klebestreifen 36 im seitlichen Abstand voneinander aufgeklebt. Durch die einander zugewandten Längskanten der Klebestreifen 36 wird somit ein durchgehender Probenkanal 20 seitlich begrenzt, wobei durch eine entsprechende Dimensionierung eine Kapillarwirkung zum selbständigen Transport der Probenflüssigkeit erreicht wird. Über der so gebildeten ersten Transportlage 18 ist eine weitere Trägerlage 12 aufgebracht, deren Unterseite mit einer Silber-Silberchlorid-Masse als Elektrodenfläche 16 beschichtet ist, und deren Oberseite wiederum mit Gold bedampft oder besputtert ist. Auf diese Weise wird durch die beidseitig an der ersten Transportlage 18 einander gegenüberliegenden Elektrodenschichten 14, 16 ein im Bereich der Durchbrüche 32 aktives Elektrodenpaar gebildet, welches aus der Goldschicht 14 als Messelektrode und der Silber-Silberchlorid-Schicht 16 als Gegen-Referenz-Elektrode besteht. Um eine elektrische Kontaktierung der Elektroden zu ermöglichen, sind die Trägerlagen 12 seitlich versetzt zueinander so angeordnet, dass in Längsrichtung verlaufende Anschlussstreifen 38, 40 frei zugänglich überstehen.

Der vorstehend beschriebene Aufbau wiederholt sich entsprechend Fig. 3, so dass letztendlich drei gleichartige übereinander gestapelte Probenkanäle 20 entstehen, die sich gegebenenfalls nur durch das eingebrachte Reagenz 34 unterscheiden.

Zur Vereinfachung der Herstellung liegen die einzelnen Lagen zunächst als aufgerollte Bänder vor, die in einer nicht gezeigten Montagevorrichtung von Rolle zu Rolle transportiert werden und im Zuge ihres Transports stapelförmig miteinander verklebt werden. Die einzelnen Teststreifen 10 entstehen dann durch Ausstanzen von Bandabschnitten, wie es in Fig. 3 unten durch die gestrichelte Kontur veranschaulicht ist. Durch das Zerteilen werden einerseits die Einlassöffnungen 40 der Probenkanäle an der Aufgabestelle 22 zur Befüllung erzeugt und zugleich am anderen Streifenende die Entlüftungsöffnungen 42 gebildet (Fig. 1). Um das Ansaugen der Probenflüssigkeit zu erleichtern, sind die Einlassöffnungen 40 durch einen stirnseitigen Einschnitt 44 in dem Teststreifen kerbenförmig vertieft.

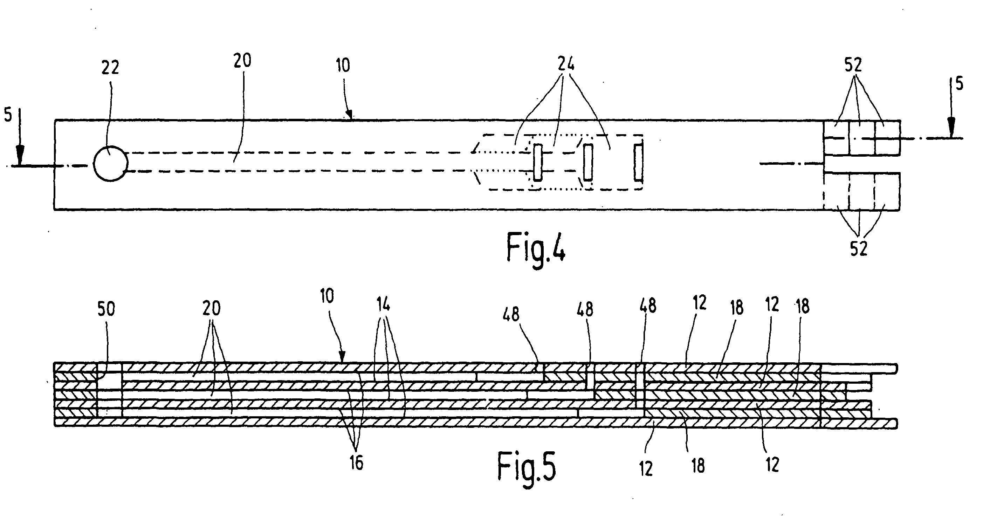

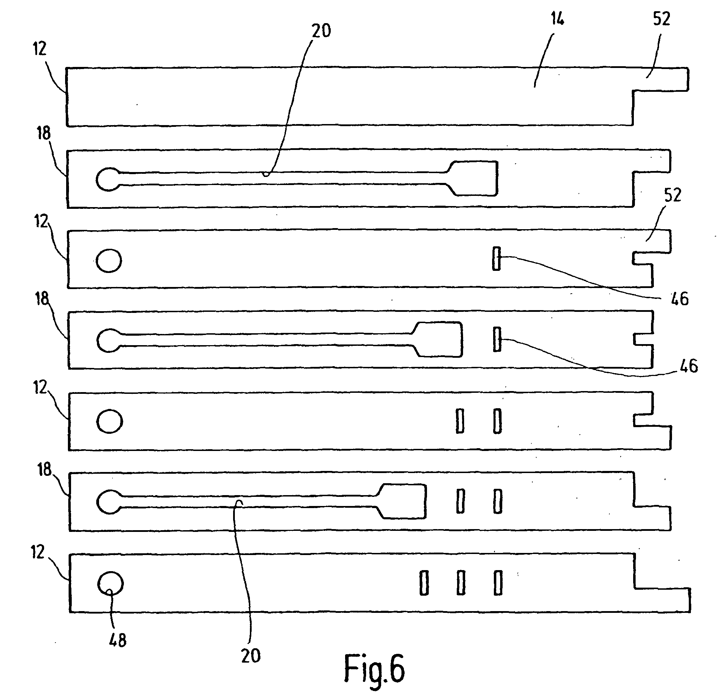

Bei den in Fig. 4 bis 8 gezeigten Ausführungsbeispielen eines Teststreifens 10 sind funktionell gleiche Teile mit gleichen Bezugszeichen wie zuvor beschrieben versehen.

Ein wesentlicher Unterschied des Ausführungsbeispiels nach Fig. 4 bis 6 besteht darin, dass in den Trägerlagen 18 der Freiraum für den Probenkanal 20 mit geschlossener Schnittlinie ausgestanzt ist. Die Streifenmontage erfolgt also nicht von der Rolle, sondern durch eine Stapel-Montage einzelner vorgestanzter Lagen. Die Messstellen 24 sind dabei durch gegebenenfalls erweiterte Endabschnitte der Probenkanäle 20 gebildet und in Streifenlängsrichtung versetzt gegeneinander angeordnet. Auf diese Weise ist es möglich, durch deckungsgleiche Durchbrüche 46 in den einzelnen Lagen Entlüftungskanäle 48 zu schaffen, welche in Stapelrichtung verlaufen und in Streifenlängsrichtung im Abstand voneinander an der Streifenoberseite münden, so dass eine Durchmischung der mit gelösten Reagenzien vermischten Probenflüssigkeit in der Nähe der Messstellen 24 zuverlässig vermieden wird.

In allen Folienlagen, mit Ausnahme der untersten Trägerfolie 12, ist im Bereich der Aufgabestelle ein kreisförmiges Loch ausgestanzt. Dadurch entsteht eine mit der Probenflüssigkeit befüllbare Einlasskammer 50, von der alle drei Probenkanäle 20 ausgehen.

Die elektrischen Anschlüsse der Elektrodenschichten 14, 16 sind am Streifenende durch versetzt herausstehende Laschen 52 der Trägerlagen 12 gebildet.

Grundsätzlich kann auch bei dieser Ausführungsform durch zusätzliche hydrophile Maskenfolien, welche direkt auf die Goldschicht 14 geklebt werden, ein flächig begrenzter Elektrodenbereich gegenüber der gegenüberliegenden Gegenelektrodenschicht 16 geschaffen werden.

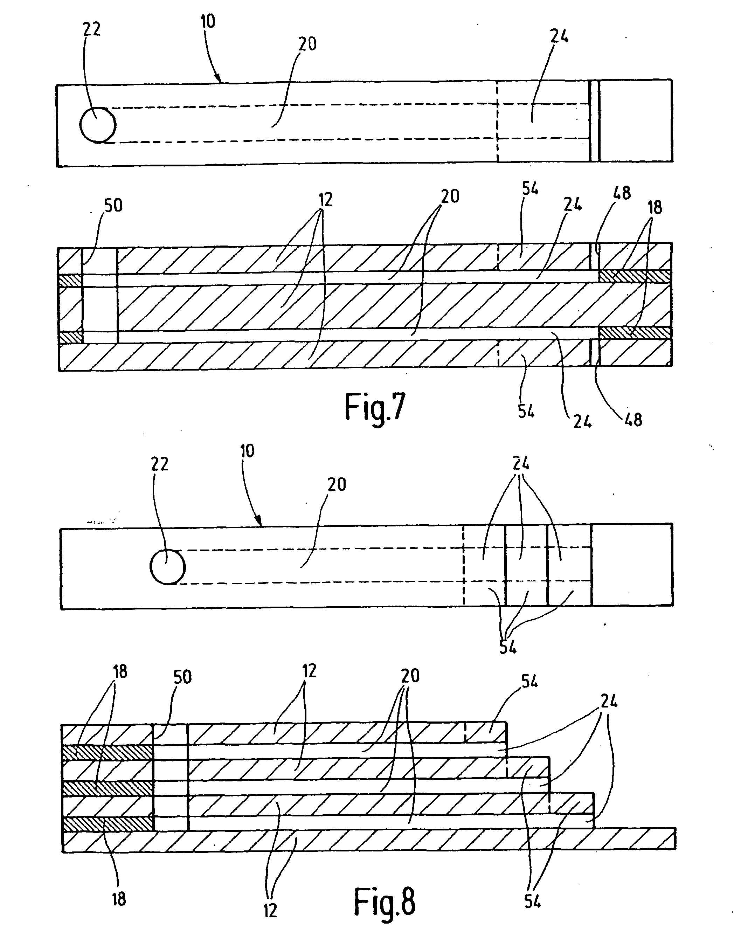

Die Ausführungsbeispiele gemäß Fig. 7 und 8 zeigen Teststreifen 10 zur optischen, insbesondere reflexionsphotometrischen Untersuchung einer Probenflüssigkeit. Zu diesem Zweck bestehen die Trägerlagen 12 zumindest im Bereich von Messfenstern 54 an den Messstellen 24 aus einem transparenten Folienmaterial. Gegebenenfalls sind in diesem Bereich von der Probenflüssigkeit aufnehmbare Nachweisreagenzien vorgehalten. Bei dem Ausführungsbeispiel nach Fig. 7 weisen die Messfenster 54 nach beiden Streifenseiten, während sie in der Ausführungsform nach Fig. 8 stufenförmig an einer Streifenseite freiliegend angeordnet sind. Auch hier sind also mehrere Probenkanäle 20 und zugeordnete Messstellen 54 stapelförmig in einem Teststreifen 10 angeordnet, um so den Nachweis mehrerer Analyten oder eine zusätzliche Funktionskontrolle zu ermöglichen.

Die Teststreifen können in an sich bekannter Weise in einem Analysegerät amperometrisch oder optisch vermessen bzw. ausgewertet werden.

Ein Einsatzgebiet der beschriebenen Teststreifen 10 ist die Analyse von Blutproben mit integrierten Kontrollen. In einem der Probenkanäle wird der eigentliche Zielparameter aus der Blutprobe erfasst. In einem weiteren Probenkanal ist ein spezielles Kontroll-Reagenz eingebracht, dass beispielsweise den zu messenden Analyten in einer vorgegebenen Menge enthält. In einem dritten Kanal ist dieser Analyt in einer davon deutlich verschiedenen Konzentration im getrockneten Reagenzfilm untergebracht. Auf diese Weise erhält man aus dem gleichen Teststreifen zwei Messwerte, zu denen die Konzentrationsdifferenz bekannt ist. Hierdurch kann sicher gestellt werden, dass der Teststreifen noch ordnungsgemäß funktioniert, und es besteht die Möglichkeit einer automatischen Kalibrierung. Spezifische Eigenschaften der Blutprobe, die unabhängig von der Parameterkonzentration den Messwert beeinflussen können, wie beispielsweise der Hämatokritgehalt und die Probentemperatur, können so kompensiert werden.

Auch für elektrochemische Substrat-Enzym-Sensoren zur Bestimmung der Blutgerinnungsparameter lassen sich auf diese Weise "On-Board-Controls" realisieren, indem in zusätzlichen Kontroll-Probenkanälen aus der aufgeteilten Blutprobe durch geeignete Reagenzien eine schnelle Gerinnung und eine längere Gerinnungszeit erzwungen werden. Auch dabei kann aus dem Verhältnis der Kontrollwerte die Kalibrierung in Abhängigkeit von Eigenschaften der Blutprobe angepasst werden. Von besonderer Bedeutung ist hier die Sicherstellung der Funktion des einzelnen Teststreifens.

Ein weiteres Anwendungsgebiet der erfindungsgemäßen Teststreifen sind Mehr-Parameter-Tests ("Panel-Testing"), bei denen dann aus der gleichen Blutprobe in mehreren übereinander angeordneten Probenkanälen verschiedene Messparameter gleichzeitig bestimmt werden. Hierzu werden in den verschiedenen Messfeldern jeweils die geeigneten Reagenzien vorgehalten.

## Patentansprüche

1. Testvorrichtung zur Untersuchung einer, insbesondere biologischen, Probenflüssigkeit mit einem aus mehreren Flachmateriallagen (12,18,30) gebildeten Verbundkörper (10) und mindestens einem in dem Verbundkörper (10) angeordneten Probenkanal (20) zum Transport der Probenflüssigkeit von einer Aufgabestelle (22) zu einer Messstelle (24), **dadurch gekennzeichnet, dass** der Verbundkörper (10) mehrere zwischen Trägerlagen (12) stapelförmig angeordnete Transportlagen (18) zur Aufnahme jeweils eines zu einer zugehörigen Messstelle (24) führenden Probenkanals (20) aufweist , so dass mehrere übereinander liegende Probenkanäle entstehen und jede Transportlage zwischen zwei Trägerlagen angeordnet ist.

2. Testvorrichtung nach Anspruch 1, **dadurch gekennzeichnet, dass** die Probenkanäle (20) in Stapelrichtung der Transportlagen (18) gesehen vorzugsweise fluchtend, übereinander angeordnet sind.

3. Testvorrichtung nach Anspruch 1 oder 2, **dadurch gekennzeichnet, dass** die Probenkanäle (20) als durchgehender Freiraum zwischen jeweils zwei gesonderten Teilstücken (36) der Transportlagen (18) freigehalten sind.

4. Testvorrichtung nach einem der Ansprüche 1 bis 3, **dadurch gekennzeichnet, dass** die Probenkanäle (20) durch Zerteilen oder Ausstanzen der Transportlagen (18) freigeschnitten sind.

5. Testvorrichtung nach einem der Ansprüche 1 bis 4, **dadurch gekennzeichnet, dass** die Transportlagen (18) aus einem elektrisch isolierenden Folienmaterial bestehen.

6. Testvorrichtung nach einem der Ansprüche 1 bis 5, **dadurch gekennzeichnet, dass** die Trägerlagen (12) an ihren den Transportlagen (18) zugewandten Seiten mit einer Elektrodenschicht (14,16) aus einem elektrisch leitfähigen Material geometrisch strukturiert oder vollfächig beschichtet sind.

7. Testvorrichtung nach Anspruch 6, **dadurch gekennzeichnet, dass** die beidseitig an den Transportlagen (18) einander gegenüberliegenden Elektrodenschichten (14,16) im Bereich der Messstellen (24) ein Elektrodenpaar zur elektrochemischen Analyse einer Probeneigenschaft bilden.

8. Testvorrichtung nach Anspruch 6 oder 7, **dadurch gekennzeichnet, dass** die einander gegenüberliegenden Elektrodenschichten (14,16) paarweise aus einem Edelmetall, vorzugsweise Gold, Platin oder Palladium als Messelektrode und einer Silber-Silberchlorid-Mischung als Gegen-Referenz-Elektrode bestehen.

9. Testvorrichtung nach einem der Ansprüche 6 bis 8, **dadurch gekennzeichnet, dass** die Elektrodenschichten (14,16) eine randseitig über eine angrenzende Transportlage (18) überstehende Anschlusspartie (38,40) zur elektrischen Kontaktierung aufweisen.

10. Testvorrichtung nach einem der Ansprüche 1 bis 9, **dadurch gekennzeichnet, dass** die Trägerlagen (12) in einem Randbereich stufenförmig gegeneinander versetzt angeordnet sind.

11. Testvorrichtung nach einem der Ansprüche 6 bis 10, **dadurch gekennzeichnet, dass** die Transportlagen (18) jeweils gegenüber mindestens einer angrenzenden Elektrodenschicht (14,16) durch eine elektrisch isolierende, vorzugsweise hydrophile Folienmaske (30) getrennt sind, und dass die Folienmaske (30) im Bereich des Probenkanals (20) Durchbrüche (32) zur Bildung von strukturierten Messfeldern aufweist.

12. Testvorrichtung nach einem der Ansprüche 1 bis 11, **dadurch gekennzeichnet, dass** im Bereich der Messstellen (24) von der Probenflüssigkeit aufnehmbare Reagenzien (34) vorzugsweise als Trockensubstanz vorgehalten sind.

13. Testvorrichtung nach einem der Ansprüche 1 bis 12, **dadurch gekennzeichnet, dass** die Probenkanäle (20) zwischen der Aufgabestelle (22) und den jeweiligen Messstellen (24) als Kapillarkanäle zum selbsttätigen Kapillartransport der Probenflüssigkeit ausgebildet sind.

14. Testvorrichtung nach einem der Ansprüche 1 bis 13, **dadurch gekennzeichnet, dass** die Aufgabestelle (22) durch eine die Einlassöffnungen (40) der Probenkanäle (20) umfassende, in die Probenflüssigkeit eintauchbare Randzone des Verbundkörpers (10) gebildet ist.

15. Testvorrichtung nach einem der Ansprüche 1 bis 13, **dadurch gekennzeichnet, dass** die Aufgabestelle (22) durch eine mit den Probenkanälen (20) kommunizierende, mit der Probenflüssigkeit beaufschlagbare Ausnehmung (50) des Verbundkörpers (10) gebildet ist.

16. Testvorrichtung nach einem der Ansprüche 1 bis 15, **dadurch gekennzeichnet, dass** die Probenkanäle (20) über quer zu den Flachmateriallagen verlaufende, im seitlichen Abstand voneinander verlaufende Entlüftungskanäle (48) an der Außenseite des Verbundkörpers (10) münden.

17. Testvorrichtung nach einem der Ansprüche 1 bis 16, **dadurch gekennzeichnet, dass** an mindestens einer Kontrollstelle (26) der Probenkanäle (20) Erfassungsmittel (14,16,32) zur Befüllungskontrolle vorzugsweise mittels elektrischer Leitfähigkeitsmessungen angeordnet sind.

18. Testvorrichtung nach einem der Ansprüche 1 bis 17, **dadurch gekennzeichnet, dass** die Trägerlagen (12) zumindest im Bereich der Messstellen (24) transparente Messfenster (54) zur optischen Untersuchung der Probenflüssigkeit bilden.

19. Testvorrichtung nach einem der Ansprüche 1 bis 18, **dadurch gekennzeichnet, dass** der Verbundkörper (10) aus stapelförmig miteinander verklebten Folienstreifen (12,18) besteht.

20. Verfahren zur Herstellung einer Testvorrichtung nach einem der vorhergehenden Ansprüche, **dadurch gekennzeichnet, dass** die Flachmateriallagen als bandförmige Rollenware von Rolle zu Rolle transportiert werden und im Zuge ihres Transports stapelförmig miteinander verklebt, gegebenenfalls mit Reagenzien versehen und nachfolgend zu Teststreifen (10) zerteilt werden.

21. Verwendung einer Testvorrichtung nach einem der vorhergehenden Ansprüche zur Bestimmung unterschiedlicher Parameter der Probenflüssigkeit in jeweils zugeordneten Probenkanälen (20).

22. Verwendung einer Testvorrichtung nach einem der vorhergehenden Ansprüche zur Erfassung von Messparametern der Probenflüssigkeit und von Kontrollparametern für die Gültigkeitsprüfung oder Kalibrierung der Messparameter in jeweils zugeordneten Probenkanälen (20).

## Claims

1. Test device for analysing an especially biological sample liquid comprising a composite body (10) consisting of a plurality of layers of flat material (12, 18, 30) and at least one sample channel (20) located in the composite body (10) for transporting the sample liquid from an application site (22) to a measuring site (24), **characterized in that** the composite body (10) has a plurality of transport layers (18) arranged in a stack-like manner between support layers (12) for holding in each case a sample channel (20) leading to an associated measuring site (24), such that a plurality of sample channels lying above one another are formed and each transport layer is arranged between two support layers.

2. Test device as claimed in claim 1, **characterized in that** the sample channels (20) are arranged, preferably aligned, on top of one another in the direction in which the transport layers (18) are stacked.

3. Test device as claimed in claim 1 or 2, **characterized in that** the sample channels (20) are kept clear as a continuous free space between in each case two separate sections (36) of the transport layers (18).

4. Test device as claimed in one of the claims 1 to 3, **characterized in that** the sample channels (20) are cut free by dividing or punching out the transport layers (18).

5. Test device as claimed in one of the claims 1 to 4, **characterized in that** the transport layers (18) consist of an electrically insulating foil material.

6. Test device as claimed in one of the claims 1 to 5, **characterized in that** the sides of the support layers (12) that face the transport layers (18) are coated with an electrode layer (14, 16) made of an electrically conductive material which is geometrically structured or covers the whole area.

7. Test device as claimed in claim 6, **characterized in that** the opposing electrode layers (14, 16) on both sides of the transport layers (18) form an electrode pair in the area of the measuring sites (24) for the electro-chemical analysis of a sample property.

8. Test device as claimed in claim 6 or 7, **characterized in that** the opposing electrode layers (14, 16) are in pairs consisting of a noble metal, preferably gold, platinum or palladium as the measuring electrode and a silver-silver chloride mixture as a counter reference electrode.

9. Test device as claimed in one of the claims 6 to 8, **characterized in that** the edges of the electrode layers (14, 16) have a connecting member (38, 40) extending beyond an adjacent transport layer (18) as an electrical contact.

10. Test device as claimed in one of the claims 1 to 9, **characterized in that** the support layers (12) are displaced relative to one another in a step-like manner in an edge region.

11. Test device as claimed in one of the claims 6 to 10, **characterized in that** each of the transport layers (18) is separated from at least one adjacent electrode layer (14, 16) by an electrically insulating, preferably hydrophilic foil mask (30) and the foil mask (30) has perforations (32) in the area of the sample channel (20) for forming structured measuring fields.

12. Test device as claimed in one of the claims 1 to 11, **characterized in that** reagents (34) that can be taken up by the sample liquid are preferably provided as a dry substance in the area of the measuring sites (24).

13. Test device as claimed in one of the claims 1 to 12, **characterized in that** the sample channels (20) are designed for the automatic capillary transport of sample liquid between the site of application (22) and the respective measuring sites (24).

14. Test device as claimed in one of the claims 1 to 13, **characterized in that** the application site (22) is formed by an edge zone of the composite body (10) which encompasses the inlet openings (40) of the sample channels (20) and can be immersed in the sample liquid.

15. Test device as claimed in one of the claims 1 to 13, **characterized in that** the application site (22) is formed by a recess (50) in the composite body (10) which communicates with the sample channels (20) and can be loaded with sample liquid.

16. Test device as claimed in one of the claims 1 to 15, **characterized in that** the sample channels (20) are connected to venting channels (48) on the outer side of the composite body (10) which run crosswise to the layers of flat material and are laterally spaced from one another.

17. Test device as claimed in one of the claims 1 to 16, **characterized in that** detection means (14, 16, 32) for checking the filling preferably by means of electrical conductivity measurements are located at at least one control site (26) of the sample channels (20).

18. Test device as claimed in one of the claims 1 to 17, **characterized in that** the support layers (12) form transparent measuring windows (54) at least in the area of the measuring positions (24) for the optical examination of the sample liquid.

19. Test device as claimed in one of the claims 1 to 18, **characterized in that** the composite body (10) consists of strips of foil (12, 18) glued together in a stack-like manner.

20. Process for producing a test device as claimed in one of the previous claims, **characterized in that** the layers of flat material are transported from roll to roll as tape-like roll-material and are glued together in a stack-like manner during their transport, and are optionally provided with reagents and subsequently divided into test strips (10).

21. Use of a test device as claimed in one of the previous claims for determining different parameters of the sample liquid in respective sample channels (20).

22. Use of a test device as claimed in one of the previous claims for detecting measuring parameters of the sample liquid and of control parameters to check the validity or calibrate the measuring parameters in respective sample channels (20).

## Revendications

1. Dispositif de test pour l'analyse d'un échantillon liquide, en particulier biologique, avec un corps composite (10) formé de plusieurs couches de matériau plat (12, 18, 30) et au moins un canal d'échantillonnage (20) disposé dans le corps composite (10) pour le transport de l'échantillon liquide d'un point d'alimentation (22) à un point de mesure (24), **caractérisé par le fait que** le corps composite (10) présente plusieurs couches de transport (18) empilées entre des couches de support (12), prévues pour recevoir chacune un canal d'échantillonnage (20) conduisant à un point de mesure (24) associé, de sorte qu'il en résulte plusieurs canaux d'échantillonnage superposés et que chaque couche de transport est disposée entre deux couches de support.

2. Dispositif de test selon la revendication 1, **caractérisé par le fait que** les canaux d'échantillonnage (20) sont superposés, de préférence en alignement, dans la direction d'empilement des couches de transport (18).

3. Dispositif de test selon la revendication 1 ou 2, **caractérisé par le fait que** les canaux d'échantillonnage (20) sont réservés sous forme d'espace libre continu entre deux sections (36) séparées des couches de transport (18).

4. Dispositif de test selon l'une des revendications 1 à 3, **caractérisé par le fait que** les canaux d'échantillonnage (20) sont dégagés par séparation ou découpage des couches de transport (18).

5. Dispositif de test selon l'une des revendications 1 à 4, **caractérisé par le fait que** les couches de transport (18) sont réalisées dans un matériau en feuille électriquement isolant.

6. Dispositif de test selon l'une des revendications 1 à 5, **caractérisé par le fait que** les couches de support (12) sont structurées géométriquement ou revêtues sur toute leur surface d'une couche d'électrode (14, 16) en matériau électriquement conducteur sur leurs faces tournées vers les couches de transport (18).

7. Dispositif de test selon la revendication 6, **caractérisé par le fait que** les couches d'électrode (14, 16) se faisant face de part et d'autre des couches de transport (18) forment au niveau des points de mesure (24) une paire d'électrodes pour l'analyse électrochimique d'une caractéristique de l'échantillon.

8. Dispositif de test selon la revendication 6 ou 7, **caractérisé par le fait que** les couches d'électrode (14, 16) se faisant face soient réalisées par paires en un métal noble, de préférence en or, platine ou palladium pour l'électrode de mesure et en un mélange argent-chlorure d'argent pour la contre-électrode de référence.

9. Dispositif de test selon l'une des revendications 6 à 8, **caractérisé par le fait que** les couches d'électrode (14, 16) présentent une partie de connexion (38, 40) dépassant du bord d'une couche de transport (18) adjacente pour le contact électrique.

10. Dispositif de test selon l'une des revendications 1 à 9, **caractérisé par le fait que** les couches de support (12) sont décalées les unes par rapport aux autres en gradins au niveau d'un bord.

11. Dispositif de test selon l'une des revendications 6 à 10, **caractérisé par le fait que** les couches de transport (18) sont isolées par un masque en feuille (30) électriquement isolant, de préférence hydrophile, par rapport à au moins une couche d'électrode (14, 16) adjacente et que le masque en feuille (30) présente au niveau du canal d'échantillonnage (20) des ouvertures (32) pour la formation de champs de mesure structurés.

12. Dispositif de test selon l'une des revendications 1 à 11, **caractérisé par le fait que** des réactifs (34) absorbables par l'échantillon liquide sont appliqués, de préférence sous forme de substance sèche, au niveau des points de mesure (34).

13. Dispositif de test selon l'une des revendications 1 à 12, **caractérisé par le fait que** les canaux d'échantillonnage (20) sont réalisés sous la forme de canaux capillaires pour le transport capillaire automatique de l'échantillon liquide entre le point d'alimentation (22) et les points de mesure (24) respectifs.

14. Dispositif de test selon l'une des revendications 1 à 13, **caractérisé par le fait que** le point d'alimentation (22) est formé par une zone marginale du corps composite (10) entourant les ouvertures d'entrée (40) des canaux d'échantillonnage (20), immergeable dans l'échantillon liquide.

15. Dispositif de test selon l'une des revendications 1 à 13, **caractérisé par le fait que** le point d'alimentation (22) est formé par un évidement (50) du corps composite (10) communiquant avec les canaux d'échantillonnage (20), pouvant être rempli par l'échantillon liquide.

16. Dispositif de test selon l'une des revendications 1 à 15, **caractérisé par le fait que** les canaux d'échantillonnage (20) débouchent sur la face extérieure du corps composite (10) par des canaux de mise à l'air (48) s'étendant perpendiculairement aux couches de matériau plat, à distance latérale l'un de l'autre.

17. Dispositif de test selon l'une des revendications 1 à 16, **caractérisé par le fait que** des moyens de saisie (14, 16, 32) pour le contrôle de remplissage, de préférence par des mesures de conductivité électrique, sont disposés à au moins un point de contrôle (26) des canaux d'échantillonnage (20).

18. Dispositif de test selon l'une des revendications 1 à 17, **caractérisé par le fait que** les couches de support (12) forment des fenêtres de mesure (54) transparentes au moins au niveau des points de mesure (24) pour l'analyse optique de l'échantillon liquide.

19. Dispositif de test selon l'une des revendications 1 à 18, **caractérisé par le fait que** le corps composite (10) est composé de bandes de feuilles (12, 18) collées ensemble sous forme de pile.

20. Procédé de fabrication d'un dispositif de test selon l'une des revendications précédentes, **caractérisé par le fait que** les couches de matériau plat sont transportées de rouleau en rouleau sous forme de matériau bobiné en bande et collées ensemble sous forme de pile au cours leur transport, le cas échéant munies de réactifs et ensuite séparées en bandes de test (10).

21. Utilisation d'un dispositif de test selon l'une des revendications précédentes pour la détermination de différents paramètres de l'échantillon liquide dans des canaux d'échantillonnage (20) respectivement associés.

22. Utilisation d'un dispositif de test selon l'une des revendications précédentes pour la saisie de paramètres de mesure de l'échantillon liquide et de paramètres de contrôle pour le contrôle de validité ou le calibrage des paramètres de mesure dans des canaux d'échantillonnage (20) respectivement associés.
